# EUROPÄISCHE PATENTSCHRIFT

(11) **EP 1 601 647 B1**
(45) Veröffentlichungstag und Bekanntmachung des Hinweises auf die Patenterteilung: **08.09.2010**
(21) Anmeldenummer: 04714758.2
(22) Anmeldetag: 26.02.2004
(51) Int. Cl.: C07C 409/22

(54) **VERFAHREN ZUR HERSTELLUNG VON LÖSUNGEN VON TRIMEREN KETON-PEROXIDEN**
METHOD FOR THE PRODUCTION OF TRIMERIC KETONE PEROXIDE SOLUTIONS
PROCEDE DE PRODUCTION DE SOLUTIONS DE PEROXYDES DE CETONE TRIMERES

(30) Priorität: 28.02.2003 DE 10308891
(43) Veröffentlichungstag der Anmeldung: 07.12.2005
(73) Patentinhaber: Degussa Initiators GmbH & Co. KG, 82049 Pullach (DE)
(72) Erfinder: HÄGEL, Eberhard, 82057 Icking (DE); APPEL, Hans, 82049 Pullach (DE)
(74) Vertreter: Dey, Michael
(86) Internationale Anmeldenummer: PCT/EP2004/001920
(87) Internationale Veröffentlichungsnummer: WO 2004/076405

(56) Entgegenhaltungen:
- DE-A- 4 438 147
- GB-A- 827 511
- GB-A- 873 614
- GB-A- 912 061
- GB-A- 998 686
- GB-A- 1 108 871
- GB-A- 1 462 854
- US-A- 2 909 513
- US-A- 3 781 303
- US-A- 3 925 417
- HARDING, M. AND WHALEN, D.: "Synthesis of Hexadecanolide" IND. ENG. CHEM., PROD. RES. DEV., Bd. 14, Nr. 4, 1975, Seiten 232-239, XP002285236
- VGL. RÖMPP LEXIKON

## Beschreibung

Trimere cyclische Ketonperoxide sind bekannt. 1968 fand P. Story (JACS 90, 817 (1968)) ein Verfahren, um durch Pyrolyse aus Tricycloalkylidenperoxiden makrocyclische Kohlenwasserstoffe und Lactone herzustellen. Die Tricycloalkyliden-Peroxide sind deshalb wertvolle Ausgangsstoffe zur Darstellung dieser sonst schwer herstellbaren Verbindungsklassen.

Besonders das Tricyclohexylidenperoxid (trimeres, cyclisches Cyclohexanonperoxid) ist zur Synthese von Cyclopentadecan und Hexadecanolid - Ausgangsstoffe für Moschusriechstoffe - eine gefragte Verbindung.

GB 998 686 A beschreibt die Herstellung von monomeren und dimeren Ketonperoxiden.

DE 44 38 147 A beschreibt die Herstellung von Methylethylketonperoxid, einem monomeren Ketonperoxid.

GB 1 462 854 A offenbart die Herstellung von dimeren Ketonperoxiden.

In der DE 21 32 316 offenbart P. Story ein 2-Stufen-Verfahren zur Herstellung von Tricycloalkylidenperoxiden. Aus Cycloalkanon, Wasserstoffperoxid und Säure stellt er ein 1-Hydroperoxy-1'-hydroxy-dicycloalkylidenperoxid her und setzt dieses weiter um mit starken Säuren zur gewünschten cyclischen Verbindung. Dabei entstehen Trimeres und Dimeres etwa im Verhältnis 75 : 25 in Ausbeuten von < 60 %, bezogen auf eingesetztes Cycloalkanon.

Der Umgang mit diesen festen Peroxiden, die sehr reib- und schlagempfindlich sind, stellt ein Sicherheitsrisiko dar.

Harding und Whalen (Industrial and Engineering Chemistry, Product Research Development 1975, Vol. 14, Nr. 4, S. 232-239) publizierten eine Studie zur Herstellung von trimerem, cyclischem Cyclohexanonperoxid und untersuchten die direkte Herstellung aus Cyclohexanon und Wasserstoffperoxid ohne Isolierung von offenkettigen Zwischenprodukten. Durch Arbeiten in Lösemitteln (hochsiedend Kohlenwasserstoffe) konnten sie das Risiko der Handhabung von festen Peroxiden vermeiden. In umfangreichen Versuchen optimierten sie die Synthesebedingungen (Art und Menge des Lösemittels, der Säure und die Temperatur). Unter besten Bedingungen erhielten sie trimeres, cyclisches Cyclohexanonperoxid in 84 % Ausbeute neben 1 bis 7 %, im Durchschnitt 4 % Dimeren. Die Reaktionszeit bei ca. 20 °C betrug 22 Stunden. Als bestgeeignete Säuren erwiesen sich 70 % Perchlorsäure und konzentrierte Salzsäure. Die Verwendung von Schwefelsäure führte zu noch wesentlich längeren Reaktionszeiten.

Von der Verwendung von Salpetersäure wurde ausdrücklich abgeraten wegen der befürchteten Bildung von instabiler Persalpetersäure.

Nachteile dieses Verfahrens sind die Verwendung von 70 % Perchlorsäure (gefährlich, teuer) oder konzentrierter Salzsäure (korrosiv; mit Wasserstoffperoxid kann Chlor entstehen), der Einsatz von viel Essigsäure als Lösevermittler (250 g/Mol Cyclohexanon, Abwasserbelastung!) und die lange Reaktionszeit, die ein Batchverfahren unwirtschaftlich, ein kontinuierliches Verfahren praktisch undurchführbar macht.

Es besteht daher ein Bedarf nach der Schaffung eines Verfahrens zur Herstellung von trimeren Ketonperoxiden, welches möglichst selektiv (ohne größere Anteile an Dimeren) zu hoher Ausbeute, kurzer Reaktionszeit und möglichst wenig Abfall führt und auch eine kontinuierliche Arbeitsweise möglich macht.

Aufgabe der Erfindung ist daher die Schaffung eines Verfahrens, welches die angegebenen Vorteile aufweist.

Erfindungsgemäß wird dies erreicht durch ein Verfahren zur Herstellung von trimeren Ketonperoxiden durch Umsetzung von cyclischen oder offenkettigen Ketonen mit H₂O₂ in Gegenwart eines sauren Katalysators in einem geeigneten Lösungsmittel, welches dadurch gekennzeichnet ist, dass als Katalysator Salpetersäure verwendet wird.

Überraschend wurde gefunden, dass entgegen der Lehre von Harding und Whalen die Verwendung von Salpetersäure keinerlei Sicherheitsrisiko beinhaltet, ja dass der Einsatz von Salpetersäure, was noch überraschender ist, die Reaktionsgeschwindigkeit so erhöht, dass die Umsetzung statt in mehr als 20 Stunden schon nach 1 bis 2 Stunden abgeschlossen ist. Das gewünschte Trimere entsteht in 90 % Ausbeute, das Verhältnis Trimer : Dimer beträgt bei Reaktionstemperaturen um 15 °C 94 : 6.

Als cyclische Ketone werden solche mit 5 bis 12 Kettengliedern verwendet. Die cyclischen Ketone können mit ein oder mehreren Alkylgruppen mit 1 bis 6 C-Atomen substituiert sein. Die in Betracht kommenden offenkettigen Ketonde weisen die Formel R¹-CO-R² auf, worin R¹ und R² unabhängig voneinander je 1 bis 15 Kohlenstoffatome in der Kette enthalten können, sodass R¹ + R² maximal den Wert 30, minimal den Wert 2 haben kann. Die Ketten können ihrerseits mit Alkylgruppen von 1 bis 6 C-Atomen substituiert sein. Als cyclisches Keton wird Cyclohexanon, sowie mit 1, 2 oder 3 Alkylgruppen mit 1 bis 6 C-Atomen substituiertes Cyclohexanon sowie Cyclopentanon bevorzugt. Beispiele für geeignete cyclische Ketone der bevorzugten Gruppe sind 3,3,5-Trimethylcyclohexanon, 2-Methylcyclohexanon, 4-Methylcyclohexanon und 4-tert.-Butylcycloheyanon. Ebenfalls erwies sich Cyclododecanon als geeignet. Unter den acyclischen Ketonen werden Methylethylketon und Aceton besonders bevorzugt. Andere bevorzugte Ketone sind solche, in denen R¹ oder/und R² 1 bis 4 C-Atome geradkettig oder verzweigt aufweist, insbesondere eine tert.-Butylgruppe ist.

Die Salpetersäure wird zweckmäßig in konzentrierter Form eingesetzt, vorzugsweise als 60 bis 75 %ige Lösung. Die Menge beträgt zweckmäßig 0,3 bis 0,7 Mol, vorzugsweise 0,4 bis 0,6 Mol pro Mol Cyclohexanon. Als Lösungsmittel eignen sich flüssige Kohlenwasserstoffe, insbesondere solche mit 6 bis 20 C-Atomen, die linear oder verzweigt sein können. Beispiele für geeignete Lösungsmittel sind Isododecan sowie die unter den Handelsbezeichnungen Shellsol T Isopar M, G und E vertriebenen Kohlenwasserstoffe, sowie Aromaten wie Toluol, Xylol und Phthalatester. Insbesondere für trimeres Methylethylketonperoxid (MEKP) und trimeres Acetonperoxid (AP) eignet sich Dieselkraftstoff als Lösungsmittel. Die genannten Lösungsmittel haben eine phlegmatisierende Wirksamkeit und verringern das Explosionsrisiko der reinen Peroxide. Zur Phlegmatisierung können darüber hinaus auch feste Phlegmatisierungsmittel wie Phthalate, und Füllstoffe wie Kreide, Kaolin und Kieselsäure insbesondere Dicyclohexylphthalat verwendet werden.

Durch die Verwendung von unpolaren Kohlenwasserstoffen als Lösungsmittel kann die Kristallisation der cyclischen trimeren Ketonperoxide in reiner Form vermieden werden, was sicherheitstechnisch ein Vorteil ist. Zu diesem Zwecke haben sich insbesondere Isoalkane, z.B. Isopar G, als nützlich erwiesen.

Als Lösungsvermittler wird im Verfahren der Erfindung wie oben erwähnt vorzugsweise Essigsäure verwendet. Je nach den gewählten Reaktionsbedingungen werden dabei mit 30 bis 100 g Essigsäure pro mol eingesetztes Keton die besten Ergebnisse erzielt.

Erfindungsgemäß konnte die Menge der als Lösungsvermittler bevorzugt verwendten Essigsäure um 80 % gegenüber dem Verfahren von Harding und Whalen verringert werden (von 250 g auf 50 g/Mol Keton). Dies ist ein wesentlicher wirtschaftlicher Faktor und führt zu einer Entlastung des Abwassers von organischen Stoffen. Als Lösungsvermittler können aber anstelle von Essigsäure oder zusätzlich zu dieser auch andere Substanzen, wie z.B. Propionsäure, Acetonitril, niedere Alkohole wie Methanol, und Gemische davon verwendet werden.

Am Ende der erfindungsgemäßen Umsetzung, die wie erwähnt zweckmäßig in einem aliphatischen Kohlenwasserstoff, z.B. Isopar G, unter Rühren und Kühlen durchgeführt wird, bildet sich nach Beendigung des Rührens ein Zweiphasengemisch. In der organischen Phase liegt das gewünschte Reaktionsprodukt in Lösung vor, in der wässrigen Phase befinden sich nicht umgesetzte Ausgangsprodukte und Nebenprodukte. Die organische Lösung kann direkt für weitere Umsetzungen verwendet werden oder man kann das trimere Ketonperoxid daraus als Feststoff isolieren.

Ein weiterer Gefahrenpunkt war bei bisherigen Verfahren, dass in der nach der Reaktion abgetrennten wässrigen Phase, die noch Keton, Wasserstoffperoxid, offenkettige Ketonperoxide und die gesamte Säure enthält, leicht beim Stehenlassen das Dimer entsteht, das als unlöslicher Feststoff ausfällt und sich bei mechanischer Beanspruchung explosionsartig zersetzen kann.

Gemäß einer bevorzugten Ausführungsform des erfindungsgemäßen Verfahrens wird die gesamte Reaktionsmischung am Ende der Umsetzung daher mit einer Natriumsulfit-Lösung behandelt, wobei H₂O₂ und alle Hydroperoxide reduziert werden. Nach der Phasentrennung kann somit keine Nachreaktion mehr eintreten.

Das Wasserstoffperoxid wird zweckmäßig in konzentrierter handelsüblicher Form eingesetzt, d.h. als 60 bis 80 %ige wässrige Lösung. Insbesondere eignet sich gut handelsübliches 70 %iges H₂O₂.

Die Reaktionstemperatur wird im Bereich von 10 bis 25 °C gehalten, vorzugsweise im Bereich von 15 bis 20 °C. In diesem bevorzugten Temperaturbereich läuft die Reaktion in 1 bis 3 Stunden vollständig ab.

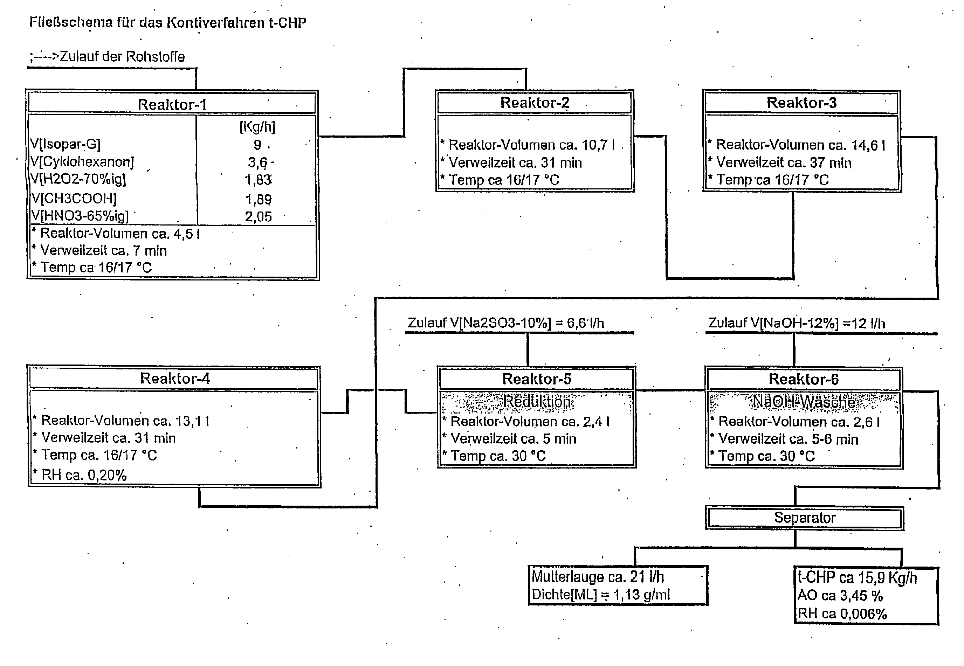

Aufgrund dieser kurzen Reaktionsdauer lässt sich das erfindungsgemäße Verfahren mit sehr guten Ergebnissen auch kontinuierlich durchführen. Für die kontinuierliche Arbeitsweise verwendet man zweckmäßig mehrere hintereinander geschaltete Reaktionsgefäße oder -behälter. Die für das vollständige Ablaufen der Umsetzung erforderliche Reaktionszeit, die wie oben erwähnt 1 bis 3 Stunden, vorzugsweise 1,5 bis 2 Stunden beträgt, erreicht man bei der kontinuierlichen Arbeitsweise durch entsprechende Abstimmung von Zahl und Größe der verschiedenen Reaktionsgefäße auf die Durchflussgeschwindigkeit. Ein Beispiel für die kontinuierliche Durchführung des erfindungsgemäßen Verfahrens unter Verwendung von sechs Reaktionsbehältern wird in Figur 1 der beigefügten Zeichnung dargestellt. Die Zahl der Reaktionsbehälter, deren Größe und die Verweilzeiten können natürlich geändert und jeweils gegebenen Bedingungen angepasst werden, wie dem Fachmann ohne Weiteres ersichtlich ist.

Die erfindungsgemäß erhaltenen Lösungen der trimeren Ketonperoxide oder die daraus gewonnenen festen Produkte eignen sich insbesondere zur Polymerisation von Monomeren, zur Heißhärtung von ungesättigten Polyesterharzen, zum Abbau von Polypropylen (PP), als Dieseladditive zur Verbesserung der Cetanzahl und Verminderung der Rußbildung (insbesondere trimeres MEKP und AP). Ferner lassen sich aus den erfindungsgemäß erhaltenen Lösungen die entsprechenden dimeren Ketonperoxide durch Erhitzen der organischen Lösung über 25 bis 50 °C in Gegenwart von Phlegmatisierungsmitteln erhalten, wobei die Explosionsgefahr der beim Erhitzen ausfallenden Dimeren auf ein annehmbares Maß verringert wird, so hergestellte dimere Ketonperoxide, insbesondere dimeres Cyclohexanonperoxid kann zur Herstellung von Caprolactam oder Anti-Malaria-Wirkstoffen eingesetzt werden.

Bevorzugt wird erfindungsgemäß der erhaltenen Lösung des trimeren Ketonperoxids nochmals Salpetersäure und Eisessig zugesetzt, auf über 25° C bis 50° C erhitzt und dimeres Ketonperoxid gewonnen.

Die folgenden Beispiele erläutern die Erfindung weiter.

### Beispiel 1

### Batchverfahren zur Herstellung einer Lösung von trimeren Cyclohexanonperoxid (CHP)

In einem 2 1-Reaktor legt man 300 g (3,06 Mol) Cyclohexanon, 150 g Essigsäure und 1000 g Isopar G (ein Isoalkan) vor und kühlt auf + 15 °C. Unter Rühren und Kühlen gibt man im Verlauf von ca. 15 Minuten 150 g (3,09 Mol) 70 % H₂O₂ zu, wobei die Temperatur unter + 20 °C gehalten wird. Anschließend gibt man in 25 - 30 Minuten unter guter Kühlung 135 g /1,39 Mol) 65 % Salpetersäure zu. Bei 15 - 18 °C wird 1,5 Stunden gerührt, dann eine Lösung von 40 g (0,15 Mol) Na₂So₃ in 360 g Wasser in 5 Minuten zugegeben, wobei man die Temperatur bis 30 °C ansteigen lässt. Man rührt noch weitere 10 Minuten, stellt den Rührer ab und trennt nach 10 Minuten die wässrige Phase ab (780 g).

Die organische Phase wird mit 500 ml 8 % Natronlauge und zwei Mal mit je 500 ml Wasser gewaschen.

Man erhält 1280 g einer farblosen Lösung mit einem Gehalt an trimerem, cyclischem Cyclohexanonperoxid von 24,3 % entsprechend 89,1 % der Theorie und 1,5 % Gehalt an dimerem, cyclischem Cyclohexanonperoxid in Isopar G gelöst.

### Beispiel 2

### Kontinuierliches Verfahren

Die Synthese des trimeren Cyclohexanonperoxids (t-CHP) läuft in einer Rekationskaskade ab, wie in der Zeichnung dargestellt. Dazu werden jeweils Cyclohexanon, Wasserstoffperoxid, Salpetersäure, Essigsäure und Isopar G gleichzeitig in den Reaktor 1 gefördert und dort unter Rühren und Kühlen auf 15°C gehalten. Bei dieser Temperatur bildet sich unter dem katalytischen Einfluss der Salpetersäure hauptsächlich das trimere CHP. Die nachfolgenden Reaktoren 2, 3, 4 mit gleicher Reaktionstemperatur dienen der Nachreaktion. Als Nebenprodukt entstehen das entsprechende dimere Cyclohexanonperoxid sowie offenkettiges hydroperoxidhaltiges CHP.

Die störenden Hydroperoxidgruppen werden im Reaktor 5 mit Natriumsulfit reduziert. Die noch in der Reaktionslösung vorhandene Säure wird im Reaktor 6 mit Natronlauge neutralisiert und mit der wässrigen Phase durch Trennung mit einem Separator entfernt.

Im Unterschied zum Batch-Verfahren von Beispiel 1 werden Salpetersäure, Isopar G, Essigsäure und Wasserstoffperoxid mit dem Cyclohexanon gleichzeitig zusammengebracht, die Reaktionsmischung wird ohne Abtrennung der Mutterlauge reduziert und neutralisiert. Auch wird etwas mehr Salpetersäure eingesetzt. Die AO Ausbeute steigt von ca. 89 % auf ca. 93 % an. Ferner kann mit höherer Konzentration gearbeitet werden.

### Beispiel 3

### Herstellung des trimeren Methylethylketonperoxids (MEKP)

Methylethylketon (350 g), Isododecan (400 g) und Eisessig (320 g) werden vorgelegt. Man gibt bei 15 bis 18 °C Salpetersäure 65 % (215 g) zu. Dann tropft man bei 14-18 °C das Wasserstoffperoxid 70 % (240 g) zu und rührt eine Stunde nach. Die wässrige Phase wird abgetrennt.

Die organische Phase wird Natriumsulfitlösung reduziert und mit verdünnter Natronlaufe und anschließend mit Wasser gewaschen.

| | |
|---|---|
| Ausbeute: | 716 g (75,4 %) |
| Gehalt: | 41 % trimer cyclisches MEKP |
| | 4 % dimer cyclisches MEKP |

### Beispiel 4

### Herstellung des dimer cyclischen Cyclohexanonperoxids

Die Synthese verläuft in zwei Stufen: Zunächst wird trimer cyclisches Cyclohexanonperoxid (tCHP) hergestellt. Das Produkt wird nicht isoliert und wird im zweiten Schritt zu reinem dCHP umgesetzt.

### Trimer cyclisches Cyclohexanonperoxid in Isododecan

Man legt Cyclohexanon (240 g), Eisessig (240 g) und Isododecan (800 g) vor und gibt bei 18 °C langsam Wasserstoffperoxid 70 % (120 g) zu. Dann tropft man bei 18 °C die Salpetersäure (110 g) zu. Man rühr eine Stunde bei 18 °C nach.

Man reduziert mit Natriuimsulfitlösung und wäscht mit verdünnter Natronlauge. Anschließend wird mit Wasser gewaschen. Die Lösung wird direkt zum dCHP umgesetzt.
Ausbeute: 1038 g; tCHP-Gehalt der Lösung: ca. 21 %

### Dimer cyclisches Cyclohexanonperoxid

Zur Lösung des tCHPs in Isododecan wird Eisessig (100 g) gegeben. Man tropft bei 30 °C Salpetersäure (85 g) hinzu und rührt zwei Stunden bei 40 °C nach. Dann kühlt man auf 25 °C ab, gibt Wasser hinzu und filtriert den gebildeten Niederschlag ab. Das Produkt wird mit Wasser gewaschen.
Ausbeute: 175 g wasserfeuchtes (ca. 90 %-iges) dCHP; GC-Gehalt: 99 %.

## Patentansprüche

1. Verfahren zur Herstellung von trimeren cyclischen Ketonperoxiden durch Umsetzung eines Ketons mit Wasserstoffperoxid in Gegenwart eines sauren Katalysators und Essigsäure als Lösevermittler in einem geeigneten Lösungsmittel,
**dadurch gekennzeichnet,**
**dass** als saurer Katalysator Salpetersäure verwendet wird.

2. Verfahren nach Anspruch 1,
**dadurch gekennzeichnet,**
**dass** als Lösungsmittel ein unpolarer Kohlenwasserstoff oder ein Gemisch davon verwendet wird.

3. Verfahren nach einem der vorhergehenden Ansprüche,
**dadurch gekennzeichnet,**
**dass** Essigsäure in einer Menge von 30 bis 100 g/mol Keton eingesetzt wird.

4. Verfahren nach einem der vorhergehenden Ansprüche,
**dadurch gekennzeichnet,**
**dass** man als Keton ein cyclisches Keton mit 5 bis 12 Kettengliedern, das mit ein oder mehreren Alkylgruppen mit 1 bis 6 C-Atomen substituiert sein kann oder ein acyclisches Keton der Formel R¹-CO-R², worin R¹ und R² unabhängig voneinander je 1 bis 15 Kohlenstoffatome in der Kette enthalten können und die Ketten durch Alkylgruppen mit 1 bis 6 C-Atomen substituiert sein können, verwendet.

5. Verfahren nach einem der vorhergehenden Ansprüche,
**dadurch gekennzeichnet,**
**dass** die Umsetzung im Temperaturbereich 10 bis 25 °C durchgeführt wird.

6. Verfahren nach einem der vorhergehenden Ansprüche,
**dadurch gekennzeichnet,**
**dass** man 60 bis 80 %iges Wasserstoffperoxid einsetzt.

7. Verfahren nach einem der vorhergehenden Ansprüche,
**dadurch gekennzeichnet,**
**dass** die Umsetzung im Bereich von 10 bis 20 °C innerhalb von 2 bis 3 Stunden durchgeführt wird.

8. Verfahren nach einem der vorhergehenden Ansprüche,
**dadurch gekennzeichnet,**
**dass** die Umsetzung kontinuierlich durchgeführt wird mit mehreren hintereinandergeschalteten Reaktionsgefäßen, wobei die Gesamtaufenthaltsdauer des Reaktionsgemisches 1 bis 3 Stunden beträgt.

9. Verfahren nach einem der vorhergehenden Ansprüche,
**dadurch gekennzeichnet,**
**dass** man nach Abschluss der Umsetzung Hydroperoxygruppen durch Sulfitzusatz entfernt.

10. Verfahren nach einem der vorhergehenden Ansprüche,
**dadurch gekennzeichnet,**
**dass** man der erhaltenen Lösung des trimeren Ketonperoxids nochmals Salpetersäure und Eisessig zusetzt auf > 25 °C bis 50 °C erhitzt und dimeres Ketonperoxid gewinnt.

## Claims

1. Method for producing trimeric cyclic ketone peroxides by reacting a ketone with hydrogen peroxide in a suitable solvent in the presence of an acidic catalyst and acetic acid as the solubiliser,
**characterised in that**
nitric acid is used as the acidic catalyst.

2. Method according to claim 1,
**characterised in that**
a non-polar hydrocarbon or a mixture thereof is used as the solvent.

3. Method according to any one of the preceding claims,
**characterised in that**
acetic acid is used in an amount of 30 to 100 g/mol ketone.

4. Method according to any one of the preceding claims,
**characterised in that**
a cyclic ketone with 5 to 12 chain links which can be substituted with one or more alkyl groups having 1 to 6 C atoms or an acyclic ketone of the formula R¹-CO-R², in which R¹ and R² each independently of one another can contain 1 to 15 carbon atoms in the chain and the chains can be substituted by alkyl groups having 1 to 6 C atoms, is used as the ketone.

5. Method according to any one of the preceding claims,
**characterised in that**
the reaction is carried out in a temperature range of from 10 to 25 °C.

6. Method according to any one of the preceding claims,
**characterised in that**
60 to 80 % hydrogen peroxide is used.

7. Method according to any one of the preceding claims,
**characterised in that**
the reaction is carried out within 2 to 3 hours in a range of from 10 to 20 °C.

8. Method according to any one of the preceding claims,
**characterised in that**
the reaction is carried out continuously using a plurality of successive reaction vessels, the total residence time of the reaction mixture being 1 to 3 hours.

9. Method according to any one of the preceding claims,
**characterised in that**
hydroperoxy groups are removed by adding sulphite after completion of the reaction.

10. Method according to any one of the preceding claims,
**characterised in that**
nitric acid and glacial acetic acid are again added to the resulting solution of the trimeric ketone peroxide, which is heated to > 25 °C to 50 °C, and dimeric ketone peroxide is obtained.

## Revendications

1. Procédé pour produire des peroxydes de cétone cycliques trimères par réaction d'une cétone avec le peroxyde d'hydrogène en présence d'un catalyseur acide et d'acide acétique comme promoteur de dissolution dans un solvant approprié, **caractérisé en ce que** l'acide nitrique est utilisé comme catalyseur acide.

2. Procédé selon la revendication 1 **caractérisé en ce qu'**un hydrocarbure non polaire ou un mélange de ceux-ci est utilisé comme solvant.

3. Procédé selon l'une des revendications précédentes **caractérisé en ce que** l'acide acétique est utilisé en une quantité de 30 à 100 g/mole de cétone.

4. Procédé selon l'une des revendications précédentes **caractérisé en ce que** l'on utilise comme cétone une cétone cyclique ayant 5 à 12 chaînons, qui peut être substituée avec un ou plusieurs groupes alkyle ayant 1 à 6 atomes C, ou une cétone acyclique de formule R¹-CO-R² où R¹ et R² peuvent contenir indépendamment l'un de l'autre chacun 1 à 15 atomes de carbone dans la chaîne et les chaînes peuvent être substituées par des groupes alkyle ayant 1 à 6 atomes C.

5. Procédé selon l'une des revendications précédentes **caractérisé en ce que** la réaction est conduite dans le domaine de température de 10 à 25°C.

6. Procédé selon l'une des revendications précédentes **caractérisé en ce que** l'on utilise du peroxyde d'hydrogène à 60 à 80 %.

7. Procédé selon l'une des revendications précédentes **caractérisé en ce que** la réaction est conduite dans le domaine de 10 à 20°C en 2 à 3 heures.

8. Procédé selon l'une des revendications précédentes **caractérisé en ce que** la réaction est conduite en continu avec plusieurs récipients réactionnels branchés les uns derrière les autres, où le temps de séjour total du mélange réactionnel est 1 à 3 heures.

9. Procédé selon l'une des revendications précédentes **caractérisé en ce que** l'on retire les groupes hydroperoxy par addition de sulfite après la fin de la réaction.

10. Procédé selon l'une des revendications précédentes **caractérisé en ce que** l'on ajoute encore de l'acide nitrique et de l'acide acétique glacial à la solution de peroxyde de cétone trimère obtenue, on chauffe à > 25°C à 50°C et on obtient du peroxyde de cétone dimère.
